# EUROPEAN PATENT SPECIFICATION

(11) **EP 0 722 709 B1**
(45) Date of publication and mention of the grant of the patent: **23.05.2001**
(21) Application number: 96300314.0
(22) Date of filing: 16.01.1996
(51) Int. Cl.: A61K 6/083

(54) **Fluoride releasing biomaterials**
Fluorid freisetzendes Biomaterial
Biomatériau à libération de fluorure

(30) Priority: 19.01.1995 GB 9501183
(43) Date of publication of application: 24.07.1996
(73) Proprietor: EASTMAN DENTAL INSTITUTE, London WC1X 8LD (GB)
(72) Inventor: Braden, Michael, Herts, AL5 1BD (GB); Patel, Mangala Prakesh, London NW7 3EX (GB); Pearson, Gavin John, Flinstones, Nr. Reading, RG8 8RU (GB)
(74) Representative: Allard, Susan Joyce

(56) References cited:
- EP-A- 0 356 868
- US-A- 4 001 483
- US-A- 4 264 489
- US-A- 4 282 140
- US-A- 5 034 433
- US-A- 5 171 763
- CHEMICAL ABSTRACTS, vol. 103, no. 6, 12 August 1985 Columbus, Ohio, US; abstract no. 42608, ALEKSIEVA, K. ET AL: "Comparative study of the effect of the kind and quantity of fluorides (fillers) on the properties of Duracryl plastics (laboratory studies)" XP002002608 & STOMATOLOGIYA (SOFIA) (1985), 67(1), 29-32 CODEN: STMYAN;ISSN: 0491-0982, 1985,

## Description

The present invention relates to fluoride releasing biomaterials and, in particular, to fluoride releasing biomaterials for use in dental applications such as for dental coatings, dental fillings, endodontic materials and orthodontic cements.

The use of physiologically acceptable polymeric materials is well known in the preparation of biomedical appliances such as hearing aids, artificial eyes and dentures, and as bone cements and the like.

GB-A-2107341 discloses low shrinkage monomer/copolymer compositions prepared by mixing a ligand component containing tetrahydrofurfuryl methacrylate with a solid component which is polyethylmethoacrylate or a copolymer of ethyl methacrylate with methyl methacrylate, propyl methacrylate or butyl methacrylate. The linear shrinkage of the polymeric material is preferably between 0.2% and 1.0%. The materials are stated to be of particular use for hearing aids, dental bridges and crowns, dentures, contact lenses and artificial eyes, or for other biomedical applications.

GB-A-2219303 discloses a two-phase cement mixture for orthopaedic use comprising a solid phase which is polymethyl methacrylate and a liquid phase which is monomethyl methacrylate. The solid phase may have certain fluoride materials added thereto, which make fluoride ions available to the bone.

W093/09819 discloses a monomer/polymer mixture for use in the preparation of a curable composition for introduction to a site requiring tissue repair in a human or animal body. The monomer component is an acrylate or methacrylate containing a heterocyclic ring and the polymer component is a polyacrylate, polymethacrylate or an acrylate-methacrylate copolymer.

EP-A-0554890 discloses dental composite compositions which include polyalkenoic acid, a filler which provides elutable cations which are reactive with the polyalkenoic acid and fluoride ion, water, a polymerizable monomer and/or prepolymer composition, an unsaturated organic acid containing one or more polymerizable groups and a catalyst system for polymerization. The compositions may be used as cement, liner, base, restorative, pit and fissure sealants, and/or core build up materials. The glass filler material is preferably a fluoroalumino silicate glass powder.

EP-A-0449399 discloses polymerisable dental materials comprising at least one polymerisable monomer or prepolymer, a catalyst and at least one inorganic filler which is a glass powder containing 40 to 75 wt% CaO, 5 to 30 wt% B₂O₃ and 5 to 35 wt% SiO₂. The material may optionally contain other inorganic fillers such as YbF₃, to render the material radioopaque, but the YbF₃ is substantially water insoluble.

The only current fluoride releasing materials are glass ionomer (polyalkenoate) cements, such as are disclosed in GB-A-2077281 and US-A-4337186. Whilst they release fluoride, thus inhibiting caries and are adhesive to calcified tissue, their relatively low strength and solubility in oral fluids limits their use.

Room temperature polymerising poly(methyl methacrylate) systems cannot be used directly in the mouth because of mucosal irritation. Also they have well documented disadvantages as orthopaedic bone cements, such as a high exotherm, hypotensive effects during surgery and brittleness. Furthermore, the low water uptake severely restricts fluoride release such as these materials. A material of this type is disclosed in Chemical Abstracts, Vol. 103, No.6, 12th August 1985. The dimethacrylates used in restorative dental materials also have a low water uptake and are highly cross-linked, thus severely restricting fluoride release.

US-A-4264489 discloses a provisional crown and bridge resin based on poly(ethyl methacrylate) polymer powder with monomer liquids. The materials are designed for low shrinkage and may incorporate an X-ray opaque barium glass.

The present invention provides a fluoride-releasing dental composition which comprises a polymer component which is an acrylate or methacrylate polymer or copolymer and a monomer component which is a monomeric heterocyclic acrylic, a monomeric methacrylic ester, or a C₃ to C₁₆ alkyl acrylic or methacrylic ester, the said compositions containing a non-toxic, biologically acceptable metal fluoride in an amount in the range of from 0.1 to 2% by weight based on the weight of the composition.

The heterocyclic monomer component of the compositions of the present invention preferably has the general formula: 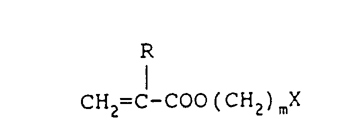 where R is a hydrogen atom or a methyl group, m is 0, 1 or 2 and X is a 3 to 6 membered heterocyclic ring. The group X is preferably a 3 to 6 membered ring containing oxygen, more preferably a tetrahydrofurfuryl group. The alkyl methacrylic ester which may be used as the monomer component is preferably a C₄ alkyl ester, more preferably an n-butyl ester for the production of rigid polymeric materials. When n is greater than 4 the methacrylate materials are compliant and less reactive.

The monomer may be admixed with one or more other monomers to control hydrophilicity, for example, hydroxyethyl methacrylate to increase hydrophilicity or isobornyl methacrylate to decrease hydrophilicity. The polymer component is preferably a methacrylate polymer, more preferably poly(ethylmethacrylate), but other polymers such as poly(methylmethacrylate), poly(hydroxyethylmethacrylate), or poly(tetrahydrofurfurylmethacrylate), or copolymers thereof, may also be employed. The copolymer component may be selected to control hydrophilicity.

The composition is suitably in the form of a mixture of finely divided solid polymer, suitably prepared by suspension polymerisation, in liquid monomer. The composition may include initially, or have added to it at the point of use, suitable activators for the curing such as free radical catalysts, e.g. peroxide/amine initiator systems. Alternatively, photoinitiators may be used, e.g. camphor quinone/tertiary amine systems which are well known in the art. Additional additives such as stabilizers and fillers and X-ray opacifying agents may also be included, if desired. These include, for example, quinone type inhibitors in the monomer, and/or inorganic fillers to increase hardness and reduce polymerisation shrinkage. In particular, hydroxyapatite may be used for this purpose and to improve biocompatability.

The ratio of the monomer to the polymer component in the compositions of the present invention may vary, depending upon the invention time to cure required and the desired consistency of the composition initially. Suitably the ratio of polymer to monomer is from 1:1 to 3:1 by weight, preferably 1.25:1 to 1.75:1. The curing will preferably occur at body temperature and curing is generally effected over a period of time of 5 to 20 minutes, preferably 10 to 15 minutes.

The non-toxic, biologically acceptable metal fluoride which is incorporated into the compositions of the present invention may be, for example, sodium, potassium, tin, calcium, zinc or bismuth fluoride, or a mixture thereof, sodium fluoride being the most preferred. The fluoride is incorporated in the compositions in an amount of from 0.1 to 2% by weight, preferably 0.5% to 1.0% by weight based on the weight of the composition. The metal fluoride will generally be provided in finely divided form and admixed with the polymer component of the compositions of the present invention.

The compositions of the present invention may be used as dental preventative or dental restoration materials. For example they may be used as dental coating materials, filling materials, endodontic materials, orthodontic cements, or the like.

It is believed that the ability of such cured compositions to release fluoride results from their ability to absorb water to an extent which enables the metal fluoride present in the compositions to diffuse into the tooth tissue.

The present invention will be further described with reference to the following Examples.

### EXAMPLE 1

Room temperature polymerising resins systems were prepared comprising powdered poly(ethylmethacrylate) containing 0.6% by weight of benzoyl peroxide as a polymerization initiator and liquid tetrahydrofurfuryl methacrylate containing 2.5% v/v of N,N'-dimethyl-p-toluidine as an activator. The polymer/monomer ratio was 1.0g/0.6ml, or appropriate multiples thereof. The samples contained 0%, 0.5% and 1% w/w sodium fluoride having an average particle size of 400 micrometres, respectively.

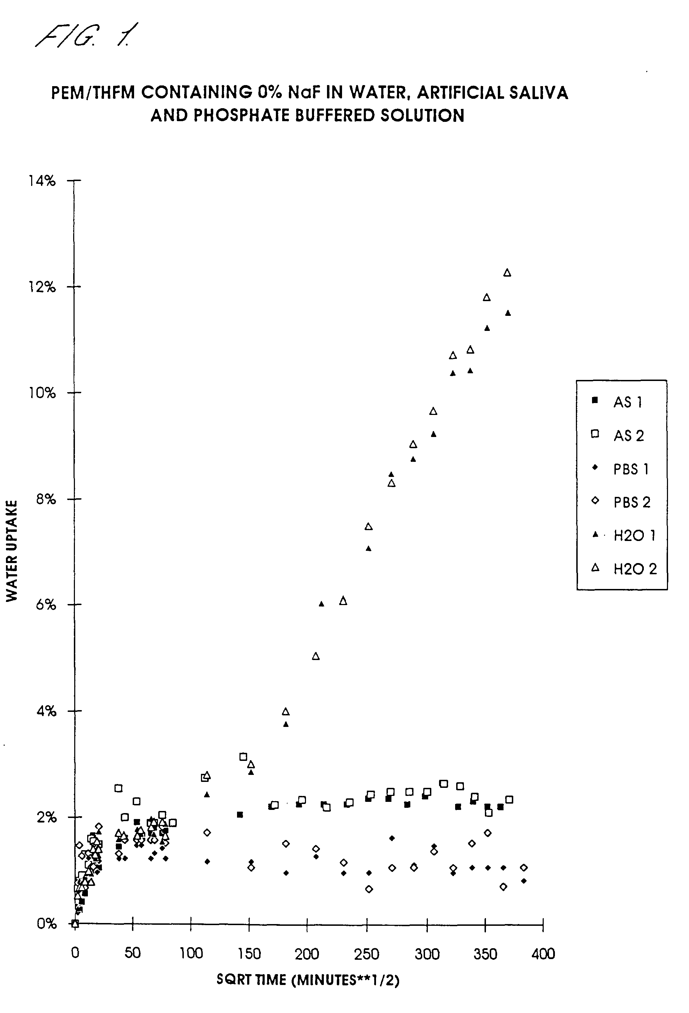

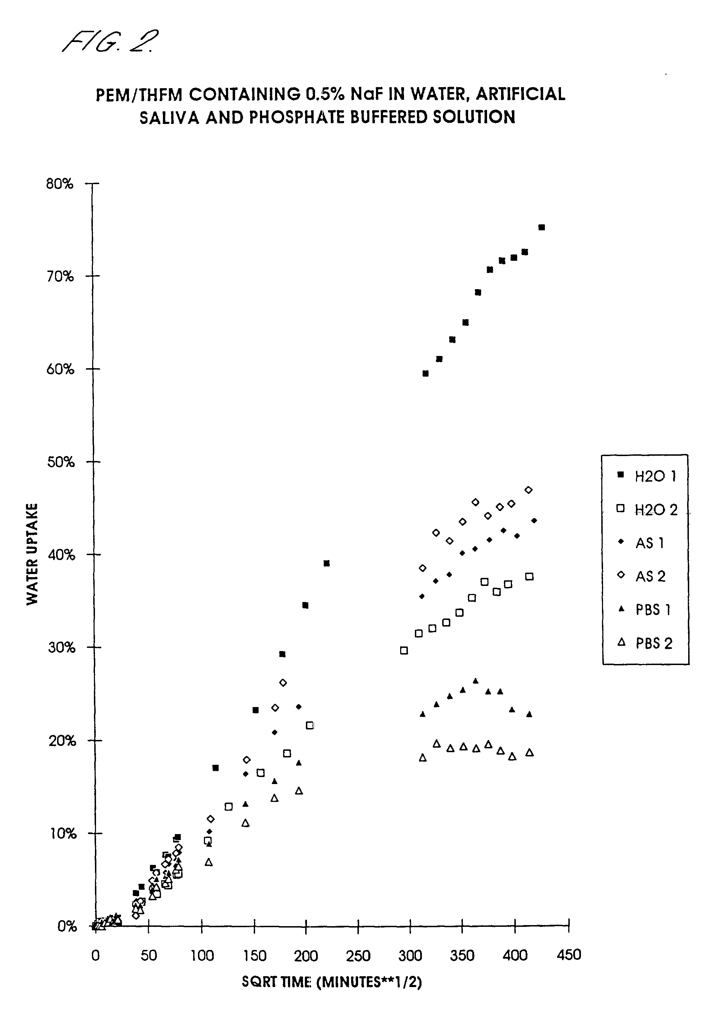

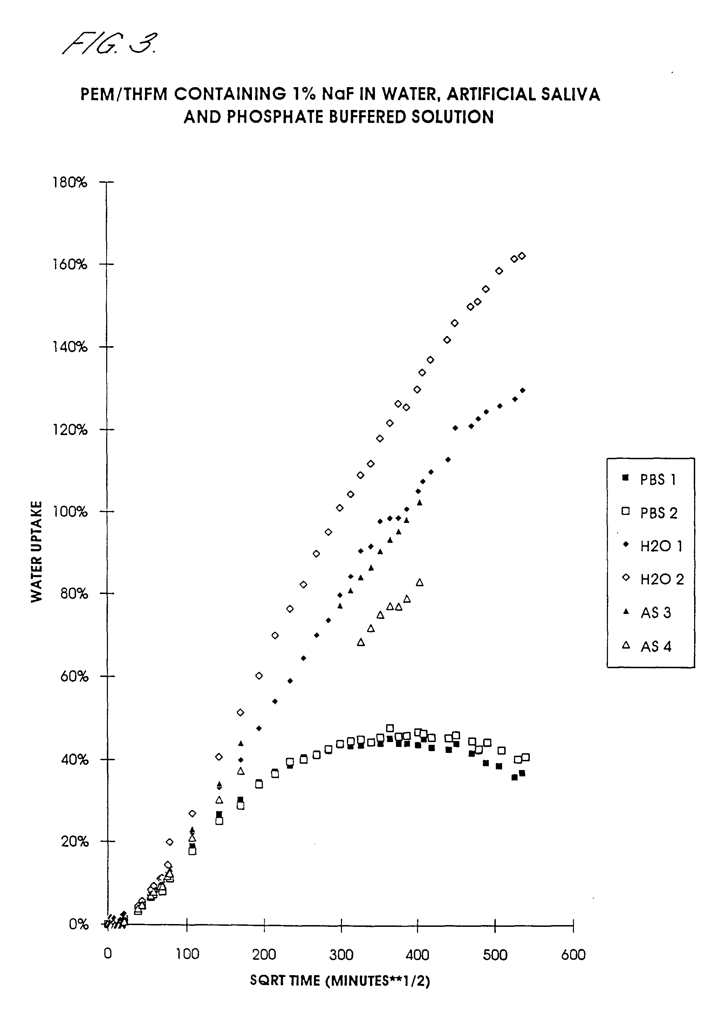

The samples were prepared in the form of discs 15mm in diameter and lmm deep, pre-weighed and immersed in distilled water, artificial saliva and phosphate buffered saline. The water uptake for the compositions containing 0%, 0.5% and 1% w/w sodium fluoride in distilled water or phosphate buffered saline are shown in Figures 1, 2 and 3.

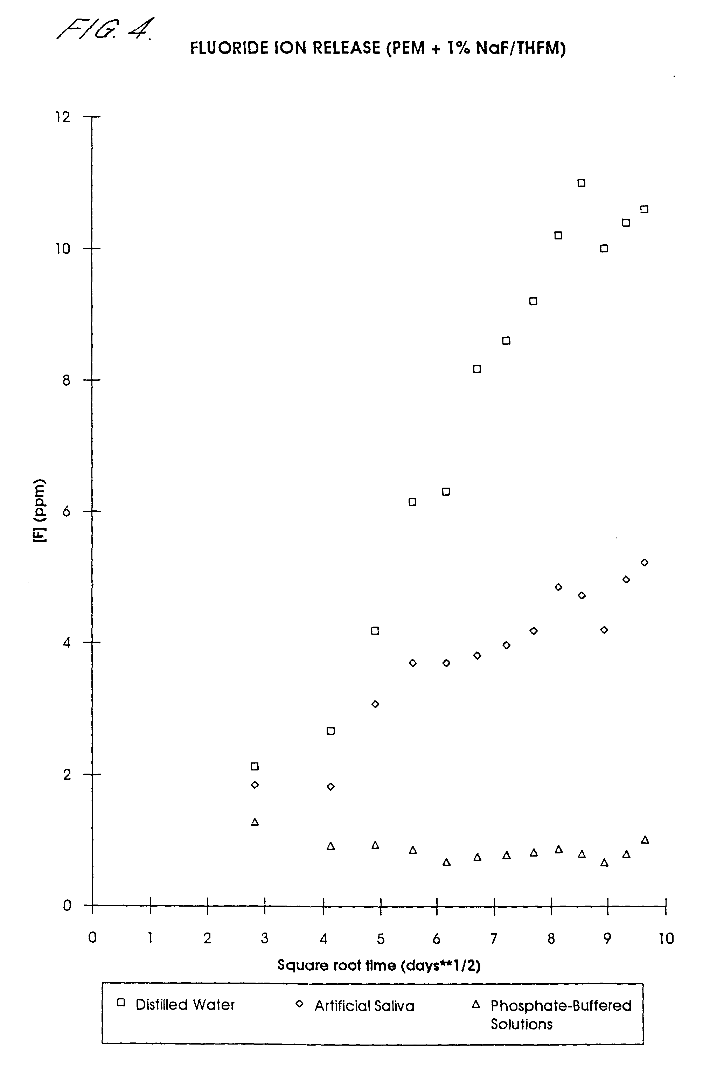

The fluoride released from the sample containing 1% w/w sodium fluoride in distilled water, artificial saliva or phosphate buffered saline was determined by the method of Tyler, J.E. and Poole, D.F.G. Arch. Oral Biol., 1989, 34, pages 995 to 998. The results are shown in Figure 4.

### EXAMPLE 2

- **A**: The procedure of Example 1 was repeated but using sodium fluoride having a particle size of 50 to 100µm obtained by grinding and sieving sodium fluoride powder.
- **B**: The procedure of Example 1 was repeated replacing tetrahydrofurfuryl methacrylate by n-butyl methacrylate and using sodium fluoride having a particle size of 50 to 150*µ*m obtained by grinding and sieving sodium fluoride powder.

The Table below gives the release data after 18 weeks for the samples from experiments A and B above immersed in water, artificial saliva and phosphate buffered saline.

**TABLE**

| **Release of Fluoride (parts per million)** | | | | |
|---|---|---|---|---|
| | **A** | | **B** | |
| % NaF | 0.5 | 1.0 | 0.5 | 1.0 |
| H₂O | 1.5 | 1.8 | 1.0 | 2.1 |
| Artificial Saliva | 1.0 | 1.3 | 0.9 | 1.9 |
| Phosphate Buffered Saline | 1.3 | 1.7 | 1.1 | 1.9 |

## Claims

1. A fluoride-releasing dental composition which comprises a polymer component which is an acrylate or methacrylate polymer or copolymer and a monomer component which is a monomeric heterocyclic acrylic ester, a monomeric heterocyclic methacrylic ester, or a C₃ to C₁₆ alkyl acrylic or methacrylic ester, the said compositions containing a non-toxic, biologically acceptable metal fluoride in an amount in the range of from 0.1 to 2% by weight based on the weight of the composition.

2. A dental composition as claimed in claim 1 wherein the heterocylic monomer component has the general formula 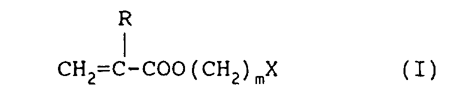 where R is a hydrogen atom or a methyl group, m is O, 1 or 2 and X is a 3 to 6 membered heterocyclic ring.

3. A dental composition as claimed in claim 2 wherein X in the monomer component is a heterocyclic ring containing oxygen.

4. A dental composition as claimed in claim 3 wherein the monomer component is tetrahydrofurfuryl methacrylate.

5. A dental composition as claimed in claim 1 wherein the monomer component is a butyl methacrylate.

6. A dental composition as claimed in any one of the preceding claims wherein the polymer component is a poly(alkylmethacrylate), preferably poly(ethylmethacrylate).

7. A dental composition as claimed in any one of the preceding claims wherein the metal fluoride is sodium fluoride.

8. A dental composition as claimed in any one of the preceding claims wherein the metal fluoride is contained in the composition in an amount of from 0.5 to 1.0% by weight.

9. A dental composition as claimed in any one of the preceding claims which additionally includes one or more filler materials.

10. A dental coating material, filling material, endodontic material or orthodontic cement which comprises a composition as claimed in any one of the preceding claims.

## Patentansprüche

1. Fluorid-freisetzende Dentalzusammensetzung, die eine Polymerkomponente, welche ein Acrylat- oder Methacrylatpolymer oder Copolymer ist und eine Monomerkomponente, welche ein monomerer heterocyclischer Acrylsäureester, ein monomerer heterocyclischer Methacrylsäureester oder ein C₃ bis C₁₆ Alkylacrylsäureester oder C₃ bis C₁₆ Alkylmethacrylsäureester ist, umfasst, wobei die Zusammensetzungen ein nicht-toxisches, biologisch verträgliches Metallfluorid in einer Menge im Bereich von 0,1 bis 2 Gew.-% enthält, bezogen auf das Gewicht der Zusammensetzung.

2. Dentalzusammensetzung nach Anspruch 1, wobei die heterocyclische Monomerkomponente die Gesamtformel 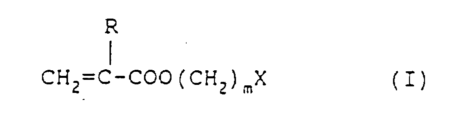 besitzt,
wobei R ein Wasserstoffatom oder eine Methylgruppe ist, m 0, 1 oder 2 ist und X ein 3- bis 6-gliedriger heterocyclischer Ring ist.

3. Dentalzusammensetzung nach Anspruch 2, wobei X in der Monomerkomponente ein heterocyclischer Ring ist, der Sauerstoff enthält.

4. Dentalzusammensetzung nach Anspruch 3, wobei die Monomerkomponente Tetrahydrofurfurylmethacrylat ist.

5. Dentalzusammensetzung nach Anspruch 1, wobei die Monomerkomponente ein Butylmethacrylat ist.

6. Dentalzusammensetzung nach einem der vorhergehenden Ansprüche, wobei die Polymerkomponente ein Poly(Alkylmethacrylat), vorzugsweise Poly(Ethylmethacrylat) ist.

7. Dentalzusammensetzung nach einem der vorhergehenden Ansprüche, wobei das Metallfluorid Natriumfluorid ist.

8. Dentalzusammensetzung nach einem der vorhergehenden Ansprüche, wobei das Metallfluorid in einer Menge von 0,5 bis 1.0 Gew.-% in der Zusammensetzung enthalten ist.

9. Dentalzusammensetzung nach einem der vorhergehenden Ansprüche, die zusätzlich ein oder mehrere Füllmaterialien enthält.

10. Dentalbeschichtungsmaterial, Füllmaterial, Wurzelfüllmaterial oder kieferorthopädischer Zement, die eine Zusammensetzung nach einem der vorhergehenden Ansprüche umfassen.

## Revendications

1. Composition dentaire libérant des fluorures, qui comprend un constituant polymère qui est un polymère ou copolymère d'acrylate ou de méthacrylate et un constituant monomère qui est un ester acrylique hétérocyclique monomère, un ester méthacrylique hétérocyclique monomère ou un ester (alkyle en C₃ à C₁₆)-acrylique ou -méthacrylique, ladite composition contenant un fluorure métallique non toxique, biologiquement acceptable, en une quantité comprise dans l'intervalle de 0,1 à 2% en poids sur la base du poids de la composition.

2. Composition dentaire suivant la revendication 1, dans laquelle le constituant monomère hétérocyclique répond à la formule générale 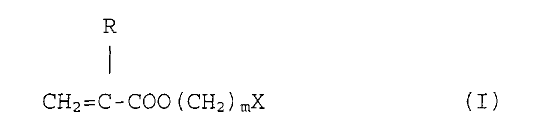 dans laquelle R représente un atome d'hydrogène ou un groupe méthyle, m est égal à 0, 1 ou 2 et X représente un noyau hétérocyclique tri- à hexagonal.

3. Composition dentaire suivant la revendication 2, dans laquelle X dans le constituant monomère est un noyau hétérocyclique contenant de l'oxygène.

4. Composition dentaire suivant la revendication 3, dans laquelle le constituant monomère est le méthacrylate de tétrahydrofurfuryle.

5. Composition dentaire suivant la revendication 1, dans laquelle le constituant monomère est le méthacrylate de butyle.

6. Composition dentaire suivant l'une quelconque des revendications précédentes, dans laquelle le constituant polymère est un poly(méthacrylate d'alkyle), de préférence le poly(méthacrylate d'éthyle).

7. Composition dentaire suivant l'une quelconque des revendications précédentes, dans laquelle le fluorure métallique est le fluorure de sodium.

8. Composition dentaire suivant l'une quelconque des revendications précédentes, dans laquelle le fluorure métallique est présent dans la composition en une quantité de 0,5 à 1,0 % en poids.

9. Composition dentaire suivant l'une quelconque des revendications précédentes, qui comprend en outre une ou plusieurs charges.

10. Matériau de revêtement dentaire, matériau d'obturation dentaire, matériau endodontique ou ciment orthodontique qui comprend une composition suivant l'une quelconque des revendications précédentes.
